# EUROPÄISCHE PATENTANMELDUNG

(11) **EP 2 614 792 A2**
(43) Veröffentlichungstag der Anmeldung: **17.07.2013**
(21) Anmeldenummer: 13150606.5
(22) Anmeldetag: 09.01.2013
(51) Int. Cl.: A61C 8/00, A61C 8/02

(54) **Medizinische Membrane, insbesondere zahnmedizinische Membrane sowie Zahnimplantat mit einer solchen Membrane**

(30) Priorität: 16.01.2012 DE 102012100326
(71) Anmelder: Neumeyer, Stefan Dr., D-93458 Eschlkam (DE)
(72) Erfinder: Neumeyer, Stefan Dr., D-93458 Eschlkam (DE)
(74) Vertreter: Graf Glück Kritzenberger

(57) **Zusammenfassung**

Medizinische, insbesondere zahnmedizinische Membrane zur Regeneration und/oder Induzierung von Gewebe, insbesondere von paradontalem Gewebe und zur Verwendung in direktem Knochen- oder Bindegewebekontakt.

## Beschreibung

Die Erfindung bezieht sich auf ein medizinisches Substrat in Form einer medizinischen Membrane gemäß Oberbegriff Patentanspruch 1 sowie auf ein Zahnimplantat gemäß Oberbegriff Patentanspruch 12. Insbesondere in der Zahnmedizin ist es vielfach erforderlich, Gewebe durch Neubildung, durch Induzierung oder durch Apposition wieder herzustellen, und zwar insbesondere, aber nicht ausschließlich auch aveoläres oder supraaveoläres Gewebe zur optimalen Integration von Dental- oder Zahnimplantaten.

Aufgabe der Erfindung ist es, eine Membrane zur verbesserten Regeneration und zur verbesserten Induzierung von Knochen- und Bindegewebe und damit insbesondere auch eine verbesserte Integration von Zahnimplantaten ermöglicht. Zur Lösung dieser Aufgabe ist eine Membran entsprechend dem Patentanspruch 1 ausgebildet. Ein Zahnimplantat ist Gegenstand des Patentanspruchs 12.

Der Ausdruck "im Wesentlichen" bzw. "etwa" bedeutet im Sinne der Erfindung Abweichungen vom jeweils exakten Wert um +/- 10%, bevorzugt um +/- 5% und/oder Abweichungen in Form von für die Funktion unbedeutenden Änderungen.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an Ausführungsbeispielen näher erläutert. Es zeigen:
- Fig. 1: in vereinfachter Darstellung ein in einer Aveole im Kiefer verankertes Zahnimplantat, mit einer das Zahnimplantat zumindest auf einer Teillänge umschließenden Membrane zwischen dem Implantatkörper und einem benachbarten Knochen- und/oder Bindegewebe;
- Fig. 2: in vereinfachter Darstellung einen Schnitt durch den Implantatkörper und die diesen umschließende Membrane;
- Fig. 3 - 7: in schematischen Darstellungen und jeweils im Schnitt verschiedene Details der Membran der Figuren 1 und 2;
- Fig. 8: in vereinfachter Darstellung einen Kieferkamm im Schnitt, zusammen mit einer zumindest zweidimensional geformten Membrane zur Regeneration und/oder Apposition von Knochengewebe.

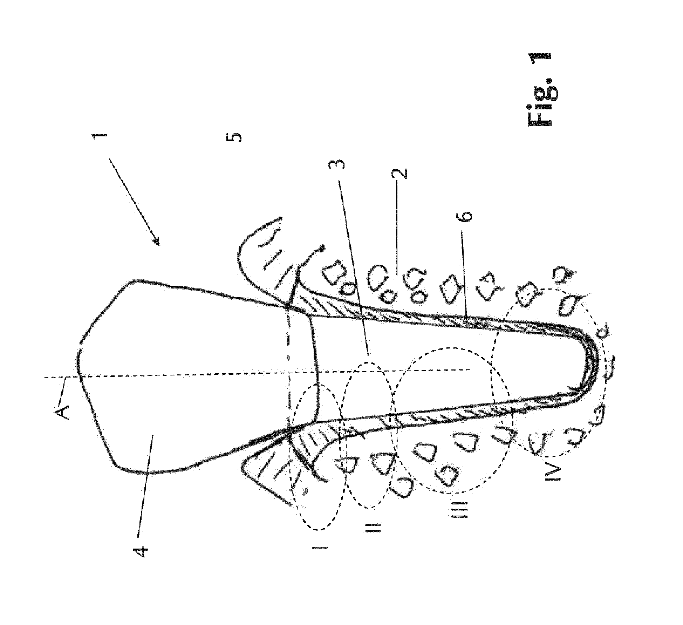

In den Figuren ist 1 ein Zahnimplantat, welches aus einem in einem Kiefer bzw. in dem dortigen Knochengewebe 2 fixierten Implantatkörper 3 z.B. aus einem metallischen Werkstoff und/oder Keramik und aus einer an die natürliche Form des ersetzten Zahns angepassten Kappe 4 aus einem hierfür geeigneten Material, beispielsweise Keramik besteht. Zumindest auf einer Teillänge ist zwischen dem Knochengewebe 2 oder einem in der Figur 1 allgemein mit 5 bezeichneten Bindegewebe eine den Implantatkörper, ggs. auch die Kappe 4 am koronalen Bereich des Implantatkörpers 3 manschettenartig umschließende Membrane 6 vorgesehen, und zwar insbesondere für eine Förderung des Einheilens des Implantates 1 bzw. für eine Verbesserung der Integration oder Einbindung des Zahnimplantats 1 in das Knochengewebe 4 und/oder das Bindegewebe 5.

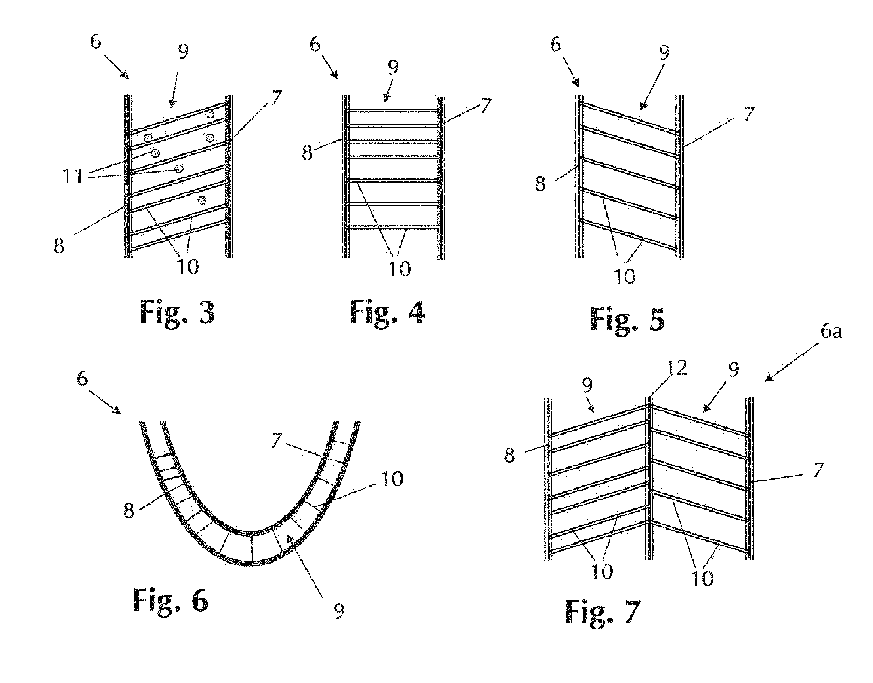

Entsprechend den Figuren 3 - 6 weist die Membrane 6 wenigstens drei unterschiedliche Strukturen oder Schichten auf, die konzentrisch oder im Wesentlichen konzentrisch zu einer Achse angeordnet sind, die der Achse A der Längserstreckung des Implantatkörpers entspricht, und zwar zumindest mit einer inneren implantatseitigen Struktur 7, einer äußeren gewebeseitigen Struktur 8 und einer dazwischen liegenden Struktur 9. Diese Strukturen sind vorzugsweise in einem nicht dargestellten porösen oder gitterartigen dreidimensionalen Trägersubstrat ausgebildet Die äußeren Strukturen sind vorzugsweise zumindest partiell mit körper- oder patienteneigenen Knochenzellen und/oder mit körper-oder patienteneigenen desmodontalen Zellen oder Strukturen und/oder Informationen versehen. Die Struktur 9 ist u.a. faserförmig, dem natürlichen Faserapparat bzw. Zahnhalterapparat nachgebildet ausgeführt, und zwar in Abhängigkeit von der Position der Membran 6 an dem Zahnimplantat oder Implantatkörper 3.

Ist die Membrane 6 beispielsweise im koronalen Bereich des Implantatkörpers und dabei beispielsweise am Übergang zu der Kappe 4 angeordnet, so ist die Struktur 9 von Faser gebildet, die von der dem Implantatkörper 3 näherliegenden oder benachbarten Struktur 7 schräg zur Achse A orientiert sind, und zwar nach Apikal, d.h. die Fasern 10 schließen mit ihrer Längserstreckung mit der Achse A einen Winkel ein, der sich zu dem unteren apikalen Ende des Implantatkörpers 3 öffnet. Außerdem weist die Struktur 9 zusätzlich zu diesen Fasern 10 weitere, bezogen auf die Achse A interzirkuläre Fasern 11 auf, d.h. das Zahnimplantat bzw. die Achse A umschließende Fasern 11, und zwar in Nachbildung des natürlichen supraalveolären Faserapparates bzw. Ligaments und zur verbesserten Integration des Zahnimplantats 1 im Zahnfleisch bzw. Bindegewinde 5, wie dies in der Figur 3 dargestellt ist.

Befindet sich die Membran 6 an der in der Figur 1 mit II bezeichneten Position mit Abstand unterhalb des koronalen Bereichs des Implantatkörpers 3, aber in einem größeren Abstand von dem apikalen Ende des Implantatkörpers 3, so sind die Fasern 10 der Struktur 9 radial oder im Wesentlichen radial zur Achse A orientiert, wie dies in der Figur 4 dargestellt ist.

Befindet sich die Membran 6 an der in der Figur 1 mit III bezeichneten Bereich des Implantatköpers 3, d.h. in weiterer Annäherung an das apikale Ende des Implantatkörpers 3, so sind entsprechend der Figur 5 die Fasern 10 der Struktur 9 radial aber schräg zur Achse A orientiert, und zwar derart, dass sie mit dieser Achse einen Winkel kleiner 90° einschließen, der sich zu dem koronalen Ende des Implantatkörpers 3 hin öffnet.

Befindet sich die Membrane 6 an dem in der Figur 1 mit IV bezeichneten Bereich, d.h. an dem apikalen Ende des Implantatkörpers 3, so sind die die Struktur 9 bildenden Faser entsprechend der Figur 6 so angeordnet, dass sie jeweils mit ihrer Längserstreckung senkrecht oder im Wesentlichen senkrecht zu der Oberfläche des apikalen und bei der dargestellten Ausführungsform abgerundeten Endes des Implantatkörpers 3 oder zu der dieses Ende umschließenden Struktur 7 orientiert sind.

Bevorzugt weist die Struktur 9 auch an wenigstens einem Bereich II - IV zusätzlich zu den Fasern 10 weitere Fasern auf, beispielsweise die Fasern 10 kreuzende und/oder die Achse A bzw. den Implantatkörper 3 umschließende interzirkuläre Fasern 11.

Bei einer bevorzugten Ausführungsform der Erfindung weist die Membrane mehr als zwei Strukturen 9 auf, wie dies in der Figur 7 für die Membrane 6a gezeigt ist, die insbesondere für die Verwendung im koronalen oder im supraalveolären Bereich des Zahnimplantates 1 geeignet ist, und zwar zur Verbesserung der Anbindung des Bindegewebes 5 an das Zahnimplantat 1 bzw. zur Förderung der Integration des Zahnimplantates 1 in das Bindegewebe oder Zahnfleisch.

Das Implantat 6a weist die Struktur 9 zweifach auf, die vorzugsweise in der vorstehend beschriebenen Weise ausgeführt ist. Zwischen den beiden Strukturen 9 ist eine zusätzliche Struktur 12 vorgesehen, von der die Fasern 10 der beiden Strukturen 9 ausgehen. Bei der dargestellten Ausführungsform sind die Fasern 10 der beiden Strukturen 9 jeweils schräg auf die mittlere Struktur 12 verlaufend orientiert. Es besteht hierbei die Möglichkeit, dass die Orientierung der Fasern 10 in beiden Strukturen 9 gleich ist oder aber wie in der Figur 7 dargestellt, die Orientierung der Fasern 10 in den Strukturen 9 unterschiedlich bzw. so ist, dass die Fasern 10 in beiden Strukturen 9 jeweils mit der Struktur 12 einen Winkel kleiner als 90° einschließen und sich dieser Winkel zu einer gemeinsamen Seite, beispielsweise nach apikal oder koronal öffnet. Auch andere Orientierungen der Fasern 10 sind möglich, und zwar vorzugsweise angepasst an die Position der Membrane 6a am Implantatkörper 3.

Es hat sich gezeigt, dass bei dem Zahnimplantat 1, welches im Gegensatz zu einem entsprechenden, natürlichen Zahn fest oder im Wesentlichen fest, d.h. nicht elastisch im Kieferknochen verankert ist, die Integration des Implantates 1 insbesondere auch in das Bindegewebe wesentlich verbessert ist, wenn zwischen der mit dem Zahnimplantat bzw. mit dem Implantatkörper 3 in geeigneter Weise, beispielweise durch Verkleben oder durch natürliches Anwachsen fest verbundenen Struktur 7 und der in das natürliche Gewebe (Knochengewebe 2, Bindegewebe 5 usw.) integrierten Struktur 8 durch die dazwischen liegende und über die Fasern 10 bewegliche Struktur 12 die entsprechenden Eigenschaften des natürlichen supraalveolären Bereichs des Zahnhalteapparates optimal nachgebildet sind, sodass mit der Membrane 6a eine optimale Integration des Zahnimplantates 1 insbesondere auch in das Bindegewebe 5 erreichbar ist.

Die Strukturen 7 - 9 und 12 bestehen beispielsweise zumindest teilweise aus einem natürlichen, d.h. körper- oder patienteneigenen Material und sind mit einer geeigneten Technik (Tissueengineering) durch Vermehren und Differenzieren eines patienteneigenen Zellmaterials erzeugt. Anstelle hiervon oder aber zusätzlich hierzu können die Strukturen 7 - 9 und 12 zumindest teilweise aus einem einem natürlichen Material nachgebildeten, vorzugsweise synthetisch nachgebildeten Material bestehen, z.B. aus einem Kollagen und/oder aus Hydroxyl-Apatit.

Angestrebt wird mit der Membrane 6 bzw. 6a die Integration des Zahnimplantates in eine der natürlichen paradontalen Struktur entsprechenden natürliche Struktur, die von dem das Zahnimplantat 1 umgebenden Gewebe ausgebildet wird, d.h. die Strukturen 7 - 9 und 12 der Membrane 6 bzw. 6a dienen primär dazu, diese Integration zu fördern. Dementsprechend besteht die Membran 6 bzw. 6a bevorzugt aus einem Material, welches - sofern es nicht das natürliche oder patienteneigene Zellmaterial ist - beim Einwachsen oder Einheilen des Zahnimplantates 1 zunehmend von dem das Implantat umgebende und/oder sich bildende natürliche bzw. aveoläre oder supraaveoläre Gewebe resorbiert wird. Ebenso besteht das Trägersubstrat, soweit vorhanden, aus einem resorbierbaren Material.

Es versteht sich, dass auch bei der Membrane 6a entsprechend der Positionierung am Implantatkörper 3 der Verlauf bzw. die Orientierung der die Strukturen 9 bildenden Fasern entsprechend gewählt ist, wie dies vorstehend für die Membrane 6 beschrieben wurde. Weiterhin können die Membrane 6 bzw. 6a so ausgeführt sein, dass sie sich über wenigstens zwei oder aber sämtliche Bereiche I - VI des Zahnimplantates 6 erstrecken oder und die Fasern 10 der Strukturen 9 dann die entsprechende Orientierung aufweisen.

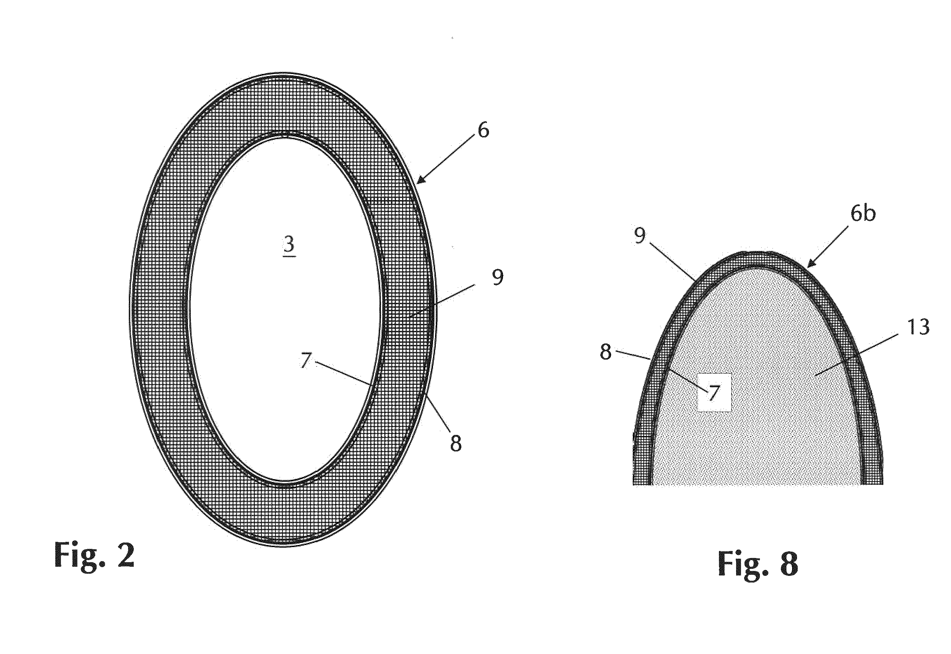

Die Figur 8 zeigt als weitere Ausführung eine Membrane 6b, die sich von der Membrane 6 im Wesentlichen nur dadurch unterscheidet, dass sie für die Verwendung zur Regeneration des Knochengewebes im Bereich eines Kieferkamms 13 ausgebildet ist. Die Membrane 6b besteht wiederum aus den beiden äußeren Strukturen 7 und 8 und der inneren Struktur 9, die von Fasern 10 und ggs. auch von diese kreuzenden Fasern 11 gebildet ist. Die Fasern 10 sind mit ihrer Längserstreckung senkrecht oder im Wesentlichen senkrecht zu den Strukturen 7 und 8 orientiert. Auch bei der Membran 6b sind die Strukturen 7, 8 und 9 wiederum bevorzugt in dem porösen oder gitterartigen Trägersubstrat ausgebildet.

Die Membranen 6, 6a und 6b bzw. deren Trägersubstrate sind beispielsweise mit einer Memoryfunktion ausgestattet, beispielsweise in der Form, dass sich das Volumen oder die Dicke der Membranen nach dem Auslösen der Memoryfunktion, beispielsweise durch Energieeintrag oder durch Aufheben einer die Memoryfunktion zunächst blockierenden Funktion, durch Strecken der Strukturen 9 zunimmt, um so z.B. sicher zu stellen, dass nach dem Einbringen des mit wenigstens einer Membran 6 und/oder 6a versehenen Implantatkörpers 3 in eine vorbereitete Aveole eine sichere Anlage der Membran gegen das umgebende Gewebe erreicht ist.

Speziell für diese Memoryfunktion sind die Fasern 10 dann solche, die beim Auslösen der Memoryfunktion in der erforderlichen Weise elastisch oder bleibend gestreckt werden können, oder aber die Fasern 10 sind zunächst gewellte Fasern, die mit dem Auslösen der Memoryfunktion zunehmend in einem gestreckten Zustand übergehen.

Die Membranen 6, 6a, 6b bzw. eventuelle Trägersubstrate dieser Membranen sind weiterhin vorzugsweise so ausgebildet, dass im Anwendungsfall eine bleibende Verformung der jeweiligen Membrane, beispielsweise durch mechanisches Strecken, Drücken usw. möglich ist, um im Anwendungsfall die Form der Membrane 6, 6a, 6b an die jeweils erforderliche Formgebung anpassen zu können. Bevorzugt besteht bei dieser Ausführungsform das Trägersubstrat aus einer Gitterstruktur, die von Fasern gebildet ist, und zwar aus einem biologisch verträglichen und vorzugsweise resorbierbaren Material.

Weiterhin kann es zweckmäßig sein, das jeweilige Trägersubstrat oder dessen Substratkörper und dabei insbesondere auch den Substratkörper aus dem resorbierbaren Material so auszubilden, dass er zunächst zellokklusiv, d.h. für natürliche Zellen nicht durchgängig ist, aber permeabel für Nährstoffe, Sauerstoff und Stoffwechselprodukte. Hierdurch ist es insbesondere möglich, die unterschiedliche Strukturen 7, 8 und 9 durch Vermehrung und Differenzierung natürlicher Zellen zu erzeugen.

Die Erfindung wurde voranstehend an Ausführungsbeispielen beschrieben. Es versteht sich, dass zahlreiche Änderungen sowie Abwandlungen möglich sind, ohne dass dadurch der der Erfindung zugrundeliegende Erfindungsgedanke verlassen wird.

### Bezugszeichenliste

- 1: Zahnimplantat
- 2: Knochengewebe
- 3: Implantatkörper
- 4: Kappe
- 5: Bindegewebe
- 6, 6a, 6b: Membrane
- 7, 8, 9: Struktur
- 10, 11: Fasern oder Ligament
- 12: Struktur
- 13: Kieferkamm
- A: Achse der Längserstreckung des Implantatkörpers 3

## Patentansprüche

1. Medizinische, insbesondere zahnmedizinische Membrane zur Regeneration und/oder Induzierung von Gewebe, insbesondere paradontalem Gewebe und zur Verwendung in direktem Knochen- oder Bindegewebekontakt,
**dadurch gekennzeichnet,**
**dass** die Membrane (6, 6a, 6b) wenigstens zweischichtig ausgebildet ist und aus zumindest zwei äußeren Strukturen (7, 8), von denen wenigstens eine zumindest partiell mit körpereigenen Knochenzellen und/oder desmodontalen Zellen, Strukturen und/oder Zellinformatianen versehen sind, sowie wenigstens einer dazwischen liegenden, eine Vielzahl von Fasern (10, 11) aufweisenden Zwischenstruktur (9) ausgebildet ist.

2. Membran nach Anspruch 1, **dadurch gekennzeichnet, dass** die Fasern (10) der wenigstens einen Zwischenstruktur (9) mit ihrer Längserstreckung senkrecht oder im Wesentlichen senkrecht und/oder in einem Winkel zu den äußeren Strukturen (7, 8) orientiert sind.

3. Membran nach Anspruch 1 oder 2, **dadurch gekennzeichnet, dass** die Fasern (10) der wenigstens einen Zwischenstruktur (9) entlang einer Achsrichtung (A) eine sich ändernde Orientierung in Bezug auf die äußeren Strukturen (7, 8) aufweisen.

4. Membran nach einem der vorhergehenden Ansprüche, **dadurch gekennzeichnet, dass** sie zwischen den äußeren Strukturen (7, 8) wenigstens zwei von Fasern (10, 11) gebildete Zwischenstrukturen (9) aufweist, zwischen denen vorzugsweise wenigstens eine weitere Struktur (12) vorgesehen ist, beispielsweise eine weitere zumindest partiell mit körpereigenen Knochenzellen und/oder desmodontalen Zellen, Strukturen und/oder Zellinformationen versehene Struktur (12).

5. Membrane nach einem der vorhergehenden Ansprüche, **dadurch gekennzeichnet, dass** die die Membran bildende Strukturen (7, 8, 9, 12) zumindest teilweise aus einem körpereigenen Material und/oder aus einem einem natürlichen Material nachgebildeten, z.B. synthetisch nachgebildeten Material bestehen, beispielsweise aus Kollagen und/oder Hydroxyl-Apatit.

6. Membrane nach einem der vorhergehenden Ansprüche, **dadurch gekennzeichnet, dass** sie zwei- oder dreidimensional geformt ist.

7. Membrane nach einem der vorhergehenden Ansprüche, **gekennzeichnet durch** wenigstens einen zumindest einen Teil der Strukturen (7, 8, 9, 12) aufnehmendes Trägersubstrat.

8. Membrane nach einem der vorhergehenden Ansprüche, **dadurch gekennzeichnet, dass** die Strukturen (7, 8, 9, 12) und/oder das Trägersubstrat aus einem resorbierbaren Material, beispielsweise aus Hydroxyl-Apatit bestehen.

9. Membrane nach einem der vorhergehenden Ansprüche, **dadurch gekennzeichnet, dass** sie oder das wenigstens eine Trägersubstrat mit einer Memoryfunktion ausgebildet ist.

10. Membrane nach einem der vorhergehenden Ansprüche, **dadurch gekennzeichnet, dass** das wenigstens eine Trägersubstrat oder ein Substratkörper für natürliche Zellen undurchgängig aber permeabel für Nährstoffe, Sauerstoff und/oder Stoffwechselprodukte ausgebildet ist.

11. Membrane nach einem der vorhergehenden Ansprüche, **dadurch gekennzeichnet, dass** zusätzlich zu den Fasern (10) weitere diese Fasern (10) kreuzende Fasern (11) vorgesehen sind.

12. Zahnimplantats mit einem Implantatkörper und einer am Implantatkörper (3) vorgesehenen Membrane (6, 6a), die wenigstens einen Teil der Außenfläche des Implantatkörpers abdeckt, **dadurch gekennzeichnet, dass** die Membrane (6, 6a) nach einem der vorhergehenden Ansprüche ausgebildet ist.
